# EUROPEAN PATENT SPECIFICATION

(11) **EP 1 180 044 B1**
(45) Date of publication and mention of the grant of the patent: **15.09.2004**
(21) Application number: 00902635.2
(22) Date of filing: 27.01.2000
(51) Int. Cl.: A61L 27/50, A61L 27/56, A61L 27/58, A61L 31/14, A61P 19/00

(54) **RESORBABLE IMPLANT**
RESORBIERBARES IMPLANTAT
IMPLANT RESORBABLE

(30) Priority: 08.02.1999 DE 19906172
(43) Date of publication of application: 20.02.2002
(73) Proprietor: Ethicon GmbH, 22851 Norderstedt (DE)
(72) Inventor: WALTHER, Christoph, D-24568 Kattendorf (DE); SCHULDT-HEMPE, Barbara, D-24576 Bad Bramstedt (DE)
(74) Representative: UEXKÜLL & STOLBERG
(86) International application number: PCT/EP2000/000622
(87) International publication number: WO 2000/047244

(56) References cited:
- US-A- 3 937 223
- US-A- 5 686 090
- US-A- 5 756 457
- MIKOS A.G., SARAKINOS G., LEITE S.M., VACANTI J., LANGER R. : "Laminated three-dimensional biodegradable foams for use in tissue engineering" BIOMATERIALS, Bd. 14, Nr. 5, 1993, Seiten 323-330, XP002137929

## Description

The invention relates to a resorbable implant which can be used in particular to develop tissues in a patient's body, e.g. bone tissue.

Bone defects which exceed a certain size are not made good by bone regeneration. Such cases require the use of bone replacement materials. Endogenous material is preferred, i.e., according to the size of the defect and the mechanical stress, spongosia, bone shavings or a bone transplant with vascular stem. However, there is not always sufficient endogenous material available, and in addition, its removal requires a second surgical procedure which gives rise to complaints or complications in up to 25% of patients.

Exogenous bone harbours the risk of transmission of disease or rejection reactions.

Artificial bone replacement materials hinder the formation of new bones, weaken the bone at the transition points, which results in a bone fracture, or themselves fail owing to material fatigue.

In 1992, Yasko et al. were the first to prove, in rats, that skeleton defects heal on administration of an artificially prepared bone protein (rhBMP-2) (J. Bone Jt. Surg. Ser. A 74(5) 659-670 (1992)). This observation was confirmed by other studies in different animal models and is also transferrable to humans. A survey can be found in Ripamonti and Duneas (Plast. Reconstr. Surg. 101 (1) 227-239 (1998)).

The fact that osteogenesis also occurs at some distance from the defect area after administration of rhBMP-2 into defects shows that a suitable support material is necessary to keep the protein at the point of application and ensure that it produces its bone-inducing effect there and nowhere else. Ideally, the support material should be completely broken down so as not to hinder bone formation. Different support materials on a polylactide/polyglycolic acid basis have already been tested.

A process for preparing artificial tissues with the help of cell interaction systems is known from WO 97/15655, in which multipliable cells of different degrees of maturation and differentiation are suspended, the cell suspension distributed in a resorbable polymer fleece and factors or components conducive to the growth and differentiation process are added to the corresponding extracellular matrix and exposed to a differentiating micromilleu. In WO 97/15655, artificial tissues are also described which consist of three-dimensional extra-cellular matrices in networkable structures, cell interaction systems to induce artificial three-dimensional tissues as well as genetically manipulated cells, which release immunosuppressive or cell-differentiating factors.

DE 43 06 661 C2 discloses a process for preparing an implant from cell structures in which cells are introduced into a three-dimensional, essentially dimensionally stable, and, according to the desired shape of the implant, pre-shaped resorbable support structure with a coherent inner surface and a small volume. Fleece materials made from polyglycolides or polylactides are suitable for this. The support structure is then encased in agarose, so that a nutrient solution can diffuse through there. By perfusing with the nutrient solution, an intercellular matrix which binds the cells to one another can develop. If necessary, a tissue-morphogenic factor can be added to the support structure, which supports cell formation and the formation of an intercellular matrix. In a variant of this process that is described in DE 44 31 598 C2, resorbable microbodies are introduced into the support structure in which factors which influence the formation of tissue are absorbed, which, for their part, are released during the gradual resorption of the microbodies. Both processes are employed in vitro.

The object of the invention is to create a possibility, with the help of which tissue and in particular differentiated tissue can be developed in a favourable way in the patient's body. The object is achieved by a resorbable implant with the features of claim 1. Advantageous designs emerge from the dependent claims.

The resorbable implant according to the invention has a structure of three-dimensional basic shape which has a density in the range of 0.05 g/cm³ to 0.20 g/cm³ and which has a porosity which produces an air permeability in the range of 1500 1/(m²s) to 7500 l/(m²s), the air permeability being measured against a test piece of 2 mm thickness, according to DIN EN ISO 9237, December 1995 edition (standard climate, 200 Pa pressure difference). The three-dimensional basic shape of the resorbable implant according to the invention is thus characterized by its density and its porosity. The porosity is quantified via the air permeability which is an easily measurable variable. The conditions for measuring air permeability are defined in DIN EN ISO 9237, December 1995 edition, for the purpose of the present invention. As this standard is geared towards areal textile structures, but the basic shape of the resorbable implant according to the invention is three-dimensional, the thickness of the test piece is agreed at 2 mm for the purpose of the present invention for the definition of unambiguous measurement instructions.

The structure of the resorbable implant consists of a synthetically prepared biocompatible material, preferably a textile yarn material, which can be broken down into molecules which can be easily metabolized and secreted by the human or animal organism. The structure exerts a tissue-conductive effect and acts e.g. osteoconductively, i.e. it guides the bone formation according to its shape. Surprisingly it has a particularly favourable effect on tissue formation if the density and the porosity of the structure are within the above-given ranges.

The structure can form a self-supporting, flexible (or else rigid) three-dimensional framework. It is however also possible that the structure has a casing (e.g. prepared from a flat knitted structure) which is filled with filling material. The shape of the resorbable implant can be pre-set directly by the structure. In one version of the resorbable implant, the structure is surrounded by a synthetic, resorbable sheath which lies against the structure and is semi-permeable or permeable. This sheath can contribute to the shaping of the resorbable implant. Shapes such as e.g. simple bodies (e.g. cuboids, tetrahedrons, cubes, cylinders, spheres, hemispheres, cylinder sections, sphere sections) can be considered for the resorbable implant, but also bodies of more complex shape which correspond to anatomical basic structures, e.g. bone sections or chondroid tissue structures.

In a preferred version, the resorbable implant includes a protein, e.g. a growth hormone which stimulates osteogenesis (preferably rhBMP-2), and can also contain patient-specific body cells. Provided with such tissue-forming endogenous (or exogenous) proteins, e.g. with osteoinductive factors such as rhBMP-2, and/or endogenous (or also exogenous) cells or cell combinations, the resorbable implant effects a tissue regeneration after being implanted in the patient. Thus e.g. a complete tissue regeneration can be achieved in the bone, even for bone defects, which, because of their size, rate as critical and would not be made good by bone regeneration if untreated throughout the patient's (whole) life. Surprisingly, the bone regeneration restricts itself to the bone defect itself; the tissue-forming factors do not stray out of the defect area. The ranges according to the invention for the density and the porosity of the structure prove to be particularly advantageous, as on the one hand a tissue formation is not hindered by a too dense or too little porous structure, while on the other hand the density is so great and the porosity so small that a guiding effect onto the growth of the tissue occurs, and a straying of cells or substances contained in the structure is prevented or at least severely hindered, even if the structure is not surrounded by a separate semi-permeable sheath.

The tissue properties and the resorption kinetics of the resorbable implant can be matched, by the choice of its material and the specification of its structure, to the different requirements, which derive from e.g. a different regeneration kinetics of the tissues. The structure allows the growth of vascular tissue as a prerequisite for the survival of the cells establishing themselves therein. Under in vivo conditions, but also in vitro, the resorbable implant creates suitable ambient conditions e.g. for the proliferation and function of osteoblasts, which, as anchorage-dependent cells, need a supporting matrix in order to survive.

The fact that, in a preferred version of the resorbable implant, the structure can be enclosed by a synthetic, resorbable sheath, which lies against the structure and is semi-permeable or permeable, has already been mentioned. Such a sheath can e.g. be designed as a tube, a pocket or a net pocket, as foil, as perforated foil or as a net. The sheath can serve to shape the resorbable implant, in particular if the structure surrounded by the sheath is not dimensionally stable, as is e.g. the case with a material like a resorbable fibre fleece. The sheath can however also facilitate a directed or shaped tissue regeneration in which the sheath acts as a guide rail. The same materials can be considered for the sheath as for the structure (see below).

In a preferred version, the structure has a monofilament portion in the range of 5% to 50%, relative to the total mass of the structure, the monofilaments preferably having a diameter in the range of 0.05 mm to 0.25 mm. The high monofilament portion has the effect that the resorbable implant is load-resistant and dimensionally stable in spite of the above-mentioned low density of the structure. According to choice of material, the resorbable implant can be thermally moulded after production in order to bring it into the desired shape. The high monofilament portion results in dimensional stability in this case also.

In a preferred version, the structure has multifilament yarns, preferably in the fineness range of e.g. 20 dtex to 200 dtex, with individual yarn finenesses of e.g. 2 dtex to 20 dtex. Multifilament yarns are more flexible than monofilaments and can be brought into an arrangement with less porosity than monofilaments.

The structure can be designed in different ways, as the following preferred versions show.

Thus the structure can have a needle fleece which e.g. consists of one component e.g. made from only one yarn type (e.g. a yarn from polyglactin 910, see below), or of several components. An example for the latter case is a sandwich structure in which different materials which are sewn together are combined.

The structure can also have a casing (e.g., a flat-knitted structure) which is filled with filling material, which casing is arranged if necessary inside the above-mentioned sheath. For example, fibre fleeces from the synthetic resorbable materials mentioned below are suitable as filling material. A conceivable composition for the structure comprises e.g. a flat knitted structure of a yarn from polyglactin 910 which is filled with fibres/threads from pre-decomposed polyglactin 910.

In a further version, the structure has various materials which shrink at different increased temperatures, the structure being brought into shape by shrinking the material shrinking at the lowest temperature. In this case, the structure can be prepared by e.g. knitting or sewing a mixture of different yarns; after that, a shrinkage of the structure takes place by an increase in temperature. The yarns are chosen so that they shrink at different temperatures, the shrinkage temperature being fixed so that only one of the yarns shrinks substantially and draws the product formed together and brings it into the desired shape. The shrinkage temperature can be chosen so that the shrinking yarn melts after shrinkage, gluing points forming in the melted material. The shrinkage temperature is preferably to be set however so that no melting occurs. This has the advantage that no large-volume gluing points form, but thin yarn bundles are present inside the structure which are better penetrated by tissue.

Preferably, the implant has poly-p-dioxanone, a glycolide/lactide copolymer, a glycolide homopolymer and/or an L-lactide homopolymer. Particularly preferred are copolymers comprising 90 parts glycolide to 10 parts L-lactide (polyglactin 910), pre-decomposed copolymers comprising polyglactin 910 and/or copolymers of 5 parts glycolide to 95 parts L-lactide. These materials have different half-life values for strength (i.e. the time after which the tensile strength of a fibre from an implanted implant has sunk to half its original value) and resorption times (i.e. the time after which the material is completely resorbed in vivo after implantation). Thus for pre-decomposed polyglactin 910 which can be prepared by radioactive irradication or by hydrolysis treatment from polyglactin 910, the half-life value for strength is 5-6 days, while the resorption time is approx. 35 days. For (non-decomposed) polyglactin 910, the corresponding values are 15-20 days and approx. 70 days. For poly-p-dioxanone, the half-life value for strength and the resorption time is 30-50 days and approx. 180 days, respectively, and for a copolymer comprising L-lactide and glycolide in the ratio of 95:5 with an inherent viscosity of 1-2 dl/g, it is 5-10 months and approx. 2-3 years, respectively. These preferred materials can be used according to the intended application for the components of the structure as well as if necessary for the sheath surrounding the structure.

The resorbable implant according to the invention can be used in different areas of medicine. The use in bone regeneration has already been mentioned. Further examples of use are for the regeneration of slow-healing tissues, e.g. tendons, bands, for an abdominal wall closure or a fascial suture or as cartilage replacement.

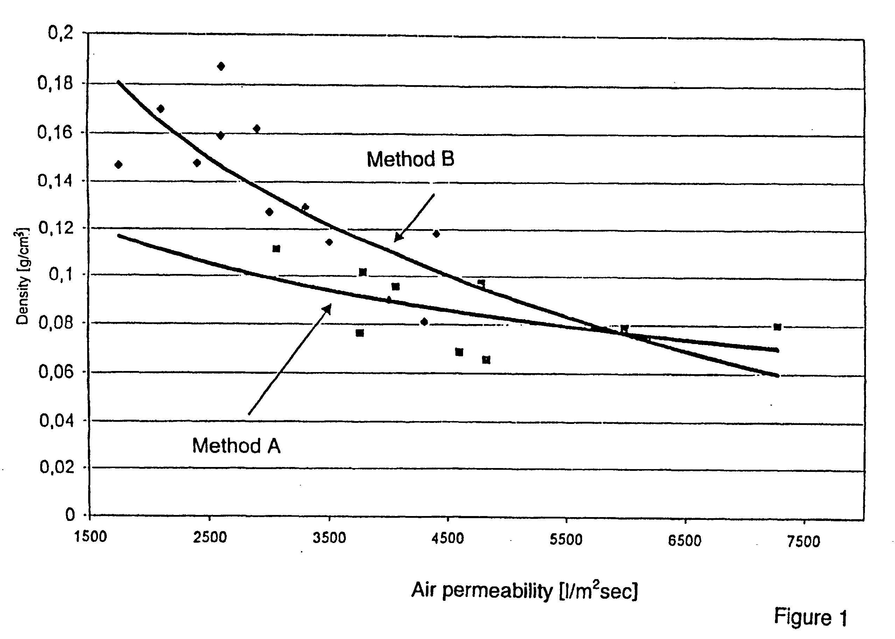

In the following it will be explained in more detail with the help of embodiments how the resorbable implant according to the invention can be prepared and used. The drawing shows in
- Figure 1: in graphical form for different versions of a resorbable implant, density as a function of air permeability.

A first group of resorbable implants is prepared by the process designated "method A" in the following. These implants each contain yarn comprising a glycolide/lactide copolymer, 90 parts glycolide and 10 parts L-lactide, as well as yarn from poly-p-dioxanone. In table 1, the number of filaments used and the corresponding yarn finenesses (in denier or den) and the mesh size applied (in mesh rows per 10 cm) in one process step (see below) are listed for twelve different versions.

**Table 1**

| Parameters of the versions according to method A | | | |
|---|---|---|---|
| No. | Glykolide/Lactide | Poly-p-dioxanone | Mesh size |
| 1 | 5 x 56 den | 1 x 60 den | 50 - 90 |
| 2 | 5 x 56 den | 1 x 60 den | 100 - 160 |
| 3 | 5 x 56 den | 1 x 30 den | 50 - 90 |
| 4 | 5 x 56 den | 1 x 30 den | 100 - 160 |
| 5 | 5 x 80 den | 1 x 60 den | 50 - 90 |
| 6 | 5 x 80 den | 1 x 30 den | 50 - 90 |
| 7 | 5 x 28 den | 1 x 60 den | 50 - 90 |
| 8 | 5 x 28 den | 1 x 60 den | 100 - 160 |
| 9 | 5 x 28 den | 1 x 30 den | 50 - 90 |
| 10 | 5 x 28 den | 1 x 30 den | 100 - 160 |
| 11 | 7 x 56 den | 1 x 60 den | 90 - 150 |
| 12 | 2 x 56 den | 1 x 60 den | 90 - 150 |

With method A, the multifilament yarns according to table 1 are jointly round-knitted. Then the resulting hose tube is flat-knitted accompanied by ribbing with the mesh size given in table 1. The flat-knitted shawl thus created is then thermally shrunk, the melting temperature of the poly-p-dioxanone not to be exceeded. At a temperature which lies under the melting temperature of the glycolide/lactide copolymer, the poly-p-dioxanone yarn draws together and brings the basic shape of the implant to its definitive form.

**Table 2**

| Parameters of the versions according to method B | | | | | |
|---|---|---|---|---|---|
| No. | Material | Recess depth | Recesses/ cm ² | No. of passages | No. layers |
| 13 | 10 x 28 den Vicryl | 14 | 100 | 4 | 2 |
| 14 | 10 x 28 den Vicryl | 14 | 100 | 9 | 2 |
| 15 | 10 x 28 den Vicryl | 14 | 100 | 2 | 2 |
| | 5 x 28 den Vicryl-r. | | | | fill. |
| 16 | 10 x 28 den Vicryl | 14 | 100 | 2 | 8 |
| 17 | 5 x 28 den Vicryl | 14 | 100 | 2 | 8 |
| 18 | 5 x 28 den Vicryl | 14 | 100 | 2 | 2 |
| 19 | 5 x 28 den Vicryl | 14 | 100 | 2 | 2 |
| | 5 x 28 den Vicryl-r. | | | | fill. |
| 20 | 10 x 28 den Vicryl-r. | 14 | 100 | 2 | 2 |
| 21 | poly-p-dioxanone | 14 | 100 | 2 | 4 |
| Vicryl-r.: Vicryl-rapid; fill.: with filling | | | | | |

A second group of resorbable implants is manufactured according to the procedure designated "method B" in the following. The implants according to method B are needle fleeces which are made with the help of a needle-felt machine using polyglactin 910 ("Vicryl") or pre-decomposed polyglactin 910 ("Vicryl-rapid") or poly-p-dioxanone by sewing flat knitted structures. Table 2 shows, for each of nine different versions, the material, the number of filaments used with the corresponding yarn finenesses (in den), the number of knitted layers of the implant as well as various sewing parameters, namely the recess depth of the needles into the knitted fabric (in mm), the number of recesses per cm² and the number of passages through the needle-felt machine. In the versions with the numbers 15 and 19, a filling is used in each case which comprises 5 x 28 denier Vicryl-rapid yarn or else 10 x 28 denier Vicryl-rapid yarn, parallel-lying strands being manufactured from ribbed round-knitted product.

For the individual versions, the density (in g/cm³) as well as the air permeability (in 1/(m ²s)) as a measure of porosity are listed in table 3. For some implants, the tearing strength (in N) is also given, in each case measured with a sample with a cross-section surface of 30 mm * 25 mm = 7.5 cm².

Figure 1 illustrates the results of table 3 in graphic form. The density is plotted as a function of air permeability for the individual versions which are drawn in as squares (method A) and as rhombuses (method.B). The fitted-in curves show that, for the two methods, different relationships result which both show a tendency towards non-linearity.

**Table 3**

| Density, air permeability and tearing strength of the versions according to method A and method B | | | |
|---|---|---|---|
| No. | Density g/cm³ | Air permeability l/(m²s) | Tearing strength N |
| 1 | 0,159 | 2100 | 127 |
| 2 | 0,068 | 4600 | 145 |
| 3 | 0,076 | 3800 | 120 |
| 4 | 0,101 | 3800 | 139 |
| 5 | 0,11 | 3100 | 169 |
| 6 | 0,08 | 7300 | 145 |
| 7 | 0,065 | 4800 | 70 |
| 8 | 0,097 | 4800 | 98 |
| 9 | 0,095 | 4100 | 68 |
| 10 | 0,079 | 6000 | 85 |
| 11 | 0,147 | 1750 | |
| 12 | 0,17 | 2100 | |
| 13 | 0,187 | 2600 | |
| 14 | 0,118 | 4440 | |
| 15 | 0,162 | 2900 | |
| 16 | 0,159 | 2600 | |
| 17 | 0,129 | 3300 | |
| 18 | 0,09 | 4000 | |
| 19 | 0,148 | 2400 | |
| 20 | 0,081 | 4300 | |
| 21 | 0,114 | 3500 | |

In the following, the use of some of the above versions is demonstrated using examples which also illustrate the properties of the implants.

### Example 1

An implant according to version no. 11 with a weight of circa 35 mg was impregnated in disk form in 150 µl 0.1% trifluoroacetic acid with 30 µg rhBMP-2 and then lyophilized. Implanted in muscle pockets in the abdominal wall of rats, it led to endochondral osteogenesis 7 days after the implantation and had fully developed to newly-formed bone tissue within 3 weeks.

### Example 2

8 mio. human cartilage cells bred in a monolayer were sown onto an implant according to version no. 12 with the dimensions 10 mm x 10 mm x 2 mm and incubated with 2-5% autologous serum in a perfusion chamber over 10 to 14 days. It was proved that the cartilage cells develop their specific matrix in the perfusion chamber within this period. The constructs comprising of implant, cartilage cells and cartilage matrix were planted under the back skin of naked mice and examined after 84 and 182 days. It was shown that the bred cartilage tissue survives.

### Example 3

Allogenic periosteal cells were obtained from rabbit bone and multiplied in a monolayer culture by a factor of 100, then sown in a suspension on an implant according to version no. 10 and incubated for a further 14 to 21 days. The implantation then took place into bone defects in the ulna of rabbits. The results were evaluated after 4 and 8 weeks and show a clear bone regeneration in the original defects. Already after 4 weeks, a homogenous bone-dense structure was observed in X-rays as a sign of clear regeneration of the bone tissue.

## Claims

1. Resorbable implant, with a structure of three-dimensional basic shape which has a density in the range of 0.05 g/cm³ to 0.20 g/cm³ and has a porosity which produces an air permeability in the range of 1500 l/(m²s) to 7500 l/(m²s), the air permeability being measured against a test piece of 2 mm thickness at 200 Pa pressure difference according to DIN EN ISO 9237, December 1995 edition.

2. Resorbable implant according to claim 1, **characterized in that** the structure is surrounded by a synthetic, resorbable casing which lies against the structure and is semi-permeable or permeable.

3. Resorbable implant according to claim 1 or 2, **characterized in that** the structure has a monofilament portion in the range from 5% to 50%, relative to the total mass of the structure, the monofilaments preferably having a diameter in the range from 0.05 mm to 0.25 mm.

4. Resorbable implant according to one of claims 1 to 3, **characterized in that** the structure has multifilament yarns.

5. Resorbable implant according to one of claims 1 to 4, **characterized in that** the structure has a needle fleece.

6. Resorbable implant according to one of claims 1 to 5, **characterized in that** the structure has a flat-knitted structure filled with filling material.

7. Resorbable implant according to one of claims 1 to 6, **characterized in that** the structure has various materials which shrink at different elevated temperatures, the structure being brought into shape by shrinkage of the material which shrinks at the lowest temperature.

8. Resorbable implant according to one of claims 1 to 7, **characterized in that** the implant has at least one of the following materials: poly-p-dioxanone, a glycolide/lactide copolymer, a glycolide homopolymer, an L-lactide homopolymer.

9. Resorbable implant according to claim 8, **characterized in that** the implant has at least one of the following materials: copolymer comprising 90 parts glycolide to 10 parts L-lactide, pre-decomposed copolymer comprising 90 parts glycolide to 10 parts L-lactide, copolymer comprising 5 parts glycolide to 95 parts L-lactide.

10. Resorbable implant according to one of claims 1 to 9, **characterized in that** the implant has a protein, preferably a growth hormone.

11. Resorbable implant according to claim 10, **characterized in that** the implant has a growth hormone which stimulates osteogenesis, preferably rhBMP-2.

12. Resorbable implant according to one of claims 1 to 11, **characterized in that** the implant has patient-specific body cells.

## Patentansprüche

1. Resorbierbares Implantat, mit einer Struktur von dreidimensionaler Grundform, die eine Dichte im Bereich von 0,05 g/cm³ bis 0,20 g/cm³ hat und die eine eine Luftdurchlässigkeit im Bereich von 1500 l/(m²s) bis 7500 l/(m²s) ergebende Porosität hat, wobei die Luftdurchlässigkeit gemäß DIN EN ISO 9237, Ausgabe Dezember 1995, an einem Prüfkörper von 2 mm Dicke bei 200 Pa Differenzdruck gemessen ist.

2. Resorbierbares Implantat nach Anspruch 1, **dadurch gekennzeichnet, daß** die Struktur von einer synthetischen, resorbierbaren Hülle umgeben ist, die an der Struktur anliegt und semipermeabel oder permeabel ist.

3. Resorbierbares Implantat nach Anspruch 1 oder 2, **dadurch gekennzeichnet, daß** die Struktur einen Monofilamentanteil im Bereich von 5% bis 50% hat, bezogen auf die Gesamtmasse der Struktur, wobei die Monofilamente vorzugsweise einen Durchmesser im Bereich von 0,05 mm bis 0,25 mm haben.

4. Resorbierbares Implantat nach einem der Ansprüche 1 bis 3, **dadurch gekennzeichnet, daß** die Struktur Multifilamentgarne aufweist.

5. Resorbierbares Implantat nach einem der Ansprüche 1 bis 4, **dadurch gekennzeichnet, daß** die Struktur ein Nadelvlies aufweist.

6. Resorbierbares Implantat nach einem der Ansprüche 1 bis 5, **dadurch gekennzeichnet, daß** die Struktur ein mit Füllmaterial gefülltes Flachgestrick aufweist.

7. Resorbierbares Implantat nach einem der Ansprüche 1 bis 6, **dadurch gekennzeichnet, daß** die Struktur verschiedene, bei unterschiedlichen erhöhten Temperaturen schrumpfende Materialien aufweist, wobei die Struktur durch Schrumpfen des bei der niedrigsten Temperatur schrumpfenden Materials in Form gebracht ist.

8. Resorbierbares Implantat nach einem der Ansprüche 1 bis 7, **dadurch gekennzeichnet, daß** das Implantat mindestens eines der folgenden Materialien aufweist: Poly-p-dioxanon, ein Glykolid/Lactid-Copolymer, ein Glykolid-Homopolymer, ein L-Lactid-Homopolymer.

9. Resorbierbares Implantat nach Anspruch 8, **dadurch gekennzeichnet, daß** das Implantat mindestens eines der folgenden Materialien aufweist: Copolymer aus 90 Teilen Glykolid auf 10 Teile L-Lactid, vorabgebautes Copolymer aus 90 Teilen Glykolid auf 10 Teile L-Lactid, Copolymer aus 5 Teilen Glykolid auf 95 Teile L-Lactid.

10. Resorbierbares Implantat nach einem der Ansprüche 1 bis 9, **dadurch gekennzeichnet, daß** das Implantat ein Protein aüfweist, vorzugsweise ein Wachstumshormon.

11. Resorbierbares Implantat nach Anspruch 10, **dadurch gekennzeichnet, daß** das Implantat ein die Knochenbildung anregendes Wachstumshormon aufweist, vorzugsweise rhBMP-2.

12. Resorbierbares Implantat nach einem der Ansprüche 1 bis 11, **dadurch gekennzeichnet, daß** das Implantat patientenspezifische Körperzellen aufweist.

## Revendications

1. Implant résorbable, avec une structure de forme de base tridimensionnelle d'une densité dans la gamme de 0,05 g/cm³ à 0,20 g/cm³ et d'une porosité qui produit une perméabilité à l'air dans la gamme de 1500 l/(m²s) à 7500 l/(m²s), la perméabilité à l'air étant mesurée par rapport un morceau test de 2 mm d'épaisseur à une différence de pression de 200 Pa conformément à la norme DIN EN ISO 9237, édition de décembre 1995.

2. Implant résorbable selon la revendication 1, **caractérisé en ce que** la structure est entourée d'une enveloppe synthétique résorbable qui est disposée tout contre la structure et est semi-perméable ou perméable.

3. Implant résorbable selon la revendication 1 ou 2, **caractérisé en ce que** la structure comporte une partie monofilamentaire dans la gamme de 5 % à 50 %, par rapport à la masse totale de la structure, les monofilaments ayant de préférence un diamètre dans la gamme de 0,05 mm à 0,25 mm.

4. Implant résorbable selon l'une quelconque des revendications 1 à 3, **caractérisé en ce que** la structure comporte des fils multifilamentaires.

5. Implant résorbable selon l'une quelconque des revendications 1 à 4, **caractérisé en ce que** la structure comporte un molleton cousu à l'aiguille.

6. Implant résorbable selon l'une quelconque des revendications 1 à 5, **caractérisé en ce que** la structure comporte une structure tricotée rectiligne comblée par un matériau de remplissage.

7. Implant résorbable selon l'une quelconque des revendications 1 à 6, **caractérisé en ce que** la structure comporte divers matériaux qui rétrécissent à différentes élévations de température, la structure étant révélée dans sa forme par le rétrécissement du matériau qui se contracte à la température la plus basse.

8. Implant résorbable selon l'une quelconque des revendications 1 à 7, **caractérisé en ce que** l'implant comporte au moins l'un des matériaux suivants : du poly-p-dioxanone, un copolymère de glycolide/lactide, un homopolymère de glycolide, un homopolymère de L-lactide.

9. Implant résorbable selon la revendication 8, **caractérisé en ce que** l'implant comporte au moins l'un des matériaux suivants : un copolymère comprenant 90 parties de glycolide pour 10 parties de L-lactide, un copolymère pré-décomposé comprenant 90 parties de glycolide pour 10 parties de L-lactide, un copolymère comprenant 5 parties de glycolide pour 95 parties de L-lactide.

10. Implant résorbable selon l'une quelconque des revendications 1 à 9, **caractérisé en ce que** l'implant comporte une protéine, de préférence une hormone de croissance.

11. Implant résorbable selon la revendication 10, **caractérisé en ce que** l'implant comporte une hormone de croissance qui stimule l'ostéogenèse, de préférence la rhBMP-2.

12. Implant résorbable selon l'une quelconque des revendications 1 à 11, **caractérisé en ce que** l'implant comporte des cellules somatiques spécifiques à un patient.
